# EUROPEAN PATENT APPLICATION

(11) **EP 2 505 223 A1**
(43) Date of publication of application: **03.10.2012**
(21) Application number: 11290162.4
(22) Date of filing: 31.03.2011
(51) Int. Cl.: A61M 21/02

(54) **Method and device for displaying images**

(71) Applicant: Alcatel Lucent, 75007 Paris (FR)
(72) Inventor: van Lier, Jan, 70825 Korntal-Münchingen (DE)
(74) Representative: Michalski Hüttermann & Partner

(57) **Abstract**

The present invention relates to a method for displaying images on an image display (14) of a display unit (12). According to the invention, the method comprises the steps:
- detecting a plurality of reference points (18, 20) within the face of at least one person looking at the image display (14), wherein the detection is performed by use of a detecting unit (16) rigidly coupled to the display unit (12);
- determining at least one movement component of a relative movement of the display unit (12) with respect to the reference points (18, 20);
- generating data of movement compensated images (34) for an at least partial compensation of the movement component with respect to the reference points (18, 20); and
- imaging the movement compensated images (34) on the image display (14). The invention relates to a corresponding device (10) for performing the method.

## Description

The present invention relates to a method for displaying images on an image display of a display unit. The invention further relates to a device comprising a display unit with an image display for displaying images, a detecting unit suitable for detecting a plurality of reference points within the face of at least one person looking at the image display and a processing unit connected to the display unit as well as the detecting unit by signal connection, wherein the detecting unit is rigidly coupled to the display unit.

A state of the art laptop computer comprises a display unit, a camera suitable for detecting reference points within the face of at least one person looking at the display unit and a processing unit connected to the display unit and the camera by signal connection. In these kinds of laptop computers, the camera and the display unit are rigidly coupled to one another in the housing part housing the display unit. Most mobile devices like laptop computers display the images like in non-mobile devices, which give rise to indisposition and motion sickness disturbing mobile application usage, e.g. in a vehicle like a train, a car or a plane.

There are already ideas for relieving motion sickness or other inconveniences caused by the use of display units in moving environment concentrating on the discrepancy between visual information and equilibrium sense but there is no solution dealing with relative short-term movements between the user and the display unit in moving environment.

It is an object of the invention to provide a method and a system for avoiding discomfort caused by use of a display unit in a moving environment due to relative short-term movements between the user and the moving display unit.

This object is achieved by the independent claims. The dependent claims detail advantageous embodiments of the invention.

The method according to the invention comprises the following steps: (i) detecting a plurality of reference points within the face of at least one person looking at the image display, wherein the detection is performed by use of a detecting unit rigidly coupled to the display unit; (ii) determining at least one movement component of a relative movement of the display unit with respect to the reference points; (iii) generating data of movement compensated images for an at least partial compensation of the movement component with respect to the reference points; and (iv) imaging the movement compensated images on the image display.

The basic idea of the invention is to sense the relative movement between the users eyes or other reference points of the face(s) and correct the displayed images in such a way that the movement compensated images give the impression to the at least one person that they are images with only little movement with respect to the person's eyes.

The relative movement is sensed by detecting the reference points within the face of the at least one person looking at the images on the image display. The detecting unit is used to perform this detection. After detecting the reference points and their positions the data of said movement compensated images are generated and send to the display unit for imaging these movement compensated images.

The relative movement may have a single motion component or may be composed from a number of motion components. The components are linear motion components or rotary motion components like a rotation, a tilt, etc. The direction of the motion components may be parallel to the imaging plane (screen plane) of the image display or perpendicular to this imaging plane of the image display. Preferably, the movement component is a movement component in a direction within a plane parallel to the screen plane of the image display.

The distance of the users face to the imaging plane is not known which would be necessary for an exact calculation of linear motion components from the detected movement of the reference points relative to the display unit by applying simple analytical geometry.

According to a preferred embodiment of the present invention, linear movement component in a plane parallel to the screen plane can be estimated by using the average inter-pupillary distance of 62 mm of adult people (US/Europe) for adjustment between sensed movement and real relative movement by the rule of proportion. Regarding the position of the detecting unit by applying simple analytical geometry for most adult people this leads to inaccuracies of only 10% or less.

According to another preferred embodiment of the present invention, the linear movement component can be calibrated by using the inter-pupillary distance of the user. This can be (i) input by the user or (ii) can be calculated like in online pupillometers via calibration with e.g. a ruler or (iii) can be calculated from an image taken when the user views the display in a defined distance to the image plane.

According to a preferred embodiment of the present invention, the movement component or at least one of the movement components is a periodically changing movement component. Especially, a maximum frequency of these compensated periodically changing movement components is limited by a first threshold (upper threshold) given by the refreshing frequency of the display device. A second threshold (lower limit) is given by a frequency where the human eye can easily follow the movement without discomfort, which is at about 2 Hz.

Preferably, the imaging of the movement compensated images at least compensates a fundamental frequency portion of the periodically changing movement component.

According to another embodiment of the present invention the movement component or at least one of the movement components is a sudden relative dislocation between the display device and the user. Especially, a minimum movement speed for non-periodical movements to be compensated is given by approximately 20 cm/s where the human eye can easily follow the movement without discomfort.

According to yet another embodiment of the present invention the image compensation due to a permanent linear or rotational movement component e.g. by a permanently changed position of the user relative to the display device is relieved with a time constant of about 2s. This is realized by an integral component to the image compensation control algorithm.

According to again another embodiment of the present invention the linear compensation for each periodic and non-periodic movement component in a plane parallel to the screen plane is limited to about 10% of the display device size to guarantee that a certain amount of the image is always visible on the display device.

According to a preferred embodiment of the present invention, a periodic or non-periodic movement perpendicular to the image plane is detected by a reduced distance between the detected reference points.

According to another preferred embodiment of the present invention, a movement or at least one movement component perpendicular to the image plane is compensated by zooming in/out the image by a factor calculated from the reference point distance change.

According to another preferred embodiment of the present invention, the detecting unit comprises at least one camera. This camera and the display unit are rigidly coupled to one another. Further on, the camera is adjusted for detecting the reference points of the face of at least one person viewing the image on the display unit. These reference points specially are reference points representing the eyes of the person(s).

According to yet another preferred embodiment of the present invention, an algorithm for face detection is used for detecting the reference points of the face. Face detection is a computer technology that determines the locations and sizes of human faces in digital images. The corresponding algorithm detects facial features and ignores other imaged objects. Many algorithms implement the face-detection task as a binary pattern-classification task. That is, the content of a given part of an image is transformed into features, after which a classifier trained on example faces decides whether that particular region of the image is a face, or not. The algorithm is implemented in the detection unit or a processing unit.

According to a preferred embodiment of the present invention, each movement compensated image is composed of the effective image content framed by a background image portion when imaged on the image display. Preferably, each effective image content of the movement compensated images uses a fraction of 80% or less than 80% of the area of the total visible image display content.

According to another embodiment of the present invention, the fraction of effective image content of the movement compensated images is adaptive taking into account the past sensed movements integrated over a certain time. The adaptive fraction is different for vertical and horizontal direction

According to yet another embodiment of the present invention, the time constants for the adaption of the fraction of effective image content of the movement compensated images is imbalanced. The time constant for increasing the size fraction of effective image content of the movement compensated images is in the range of 10 min to include sporadic movement components whereas the time constant or decreasing the size fraction of effective image content of the movement compensated images is in the range of a few seconds to cope with suddenly changed environmental conditions.

According to yet another preferred embodiment of the present invention, the relative movement is a mean relative movement, if the reference points of a plurality of persons are detected.

The invention further relates to a computer-readable medium such as a storage device, a floppy disk, CD, DVD, Blue Ray disk, or a random access memory (RAM), containing a set of instruction that causes a computer to perform an aforementioned method and a computer program product comprising a computer usable medium including computer usable program code, wherein the computer usable program code is adapted to execute the aforementioned method.

The present invention further refers to a corresponding device, especially a device for performing the aforementioned method. The processing unit of the device according to the invention is arranged for determining at least one movement component of a relative movement of the display unit with respect to the detected reference points. The processing unit is further arranged for generating data of movement compensated images for an at least partial compensation of the movement component with respect to the reference points.

According to a preferred embodiment of the present invention, the processing unit is arranged for generating data of movement compensated images for an at least partial compensation of the movement component with respect to the reference points.

According to another preferred embodiment of the present invention, the detecting unit comprises a camera. According to yet another preferred embodiment of the present invention, a face detection algorithm is implemented in the detecting unit or the processing unit.

Finally, the device preferably is a handheld device or a laptop device. These kinds of devices are for example a laptop computer, a tablet computer, an ebook reader, or any kind of other movable devices. A state of the art laptop computer already comprises the display unit, a camera suitable for detecting reference points within the face of one person or more persons looking at the image display of the display unit and a processing unit connected to the display unit as well as the camera by signal connections. In these kinds of laptop computers, the camera and the display unit are rigidly coupled to one another in one of its housing parts.

Aspects of the present invention are described below with reference to flowchart illustrations and/or block diagrams of methods, devices (systems) and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

These and other aspects of the invention will be apparent from and elucidated with reference to the embodiments described hereinafter.

In the drawings:
Fig. 1 depicts a mobile device according to a preferred embodiment of the invention formed as a laptop computer and
Fig. 2 depicts a schematic diagram illustrating the method for displaying images according to a preferred embodiment of the invention.

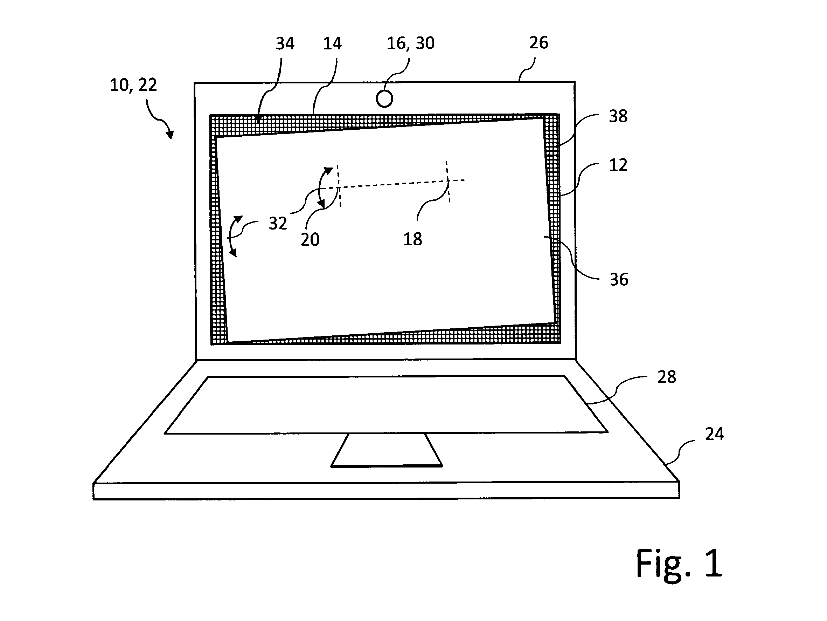

Fig. 1 depicts a device 10 comprising a display unit 12 with an image display (screen) 14 for displaying images as well as a processing unit (not shown). The device further comprises a detecting unit 16 for detecting reference points 18, 20 within a face of the person sitting in front of the image display 14 and looking at the image display 14. In this embodiment, the device is a mobile device, more precisely a laptop computer 22. The processing unit of the laptop computer 22 is located on its main board (not shown). Signal connections are connecting the processing unit to the display unit 12 and the detecting unit 16. The laptop computer 22 shows two main housing parts 24, 26 connected by a hinge. The first housing part 24 is a base of the laptop computer 22 and carries a keyboard 28 and the main board. The second housing part 26 carries the display unit 12 and a detecting unit 16 being a camera 30. Therefore, the detecting unit 16 and the display unit 12 are mechanically coupled to one another.

Fig. 1 further shows two detected reference points 18, 20 being the eye positions of the person in front of the laptop computer 22. While the first reference point 18 is fixed, the second reference point 20 is moving with respect to the camera 30 and the first reference point 18. The corresponding periodically movement (double arrow 32) of the face has at least a rotational component in a plane parallel to the imaging plane of the image display 14.

The processing unit of the system 10 is arranged for determining this at least one movement component of a relative movement of the display unit 12 with respect to the detected reference points 18, 20. The processing unit further on is arranged for generating data of movement compensated images compensating the determined movement component or movement components by moving the effective image content 36 of the present movement compensated image 34 with respect to previously presented movement compensated images.

In Fig. 1 the image display 14 displays a movement compensated image 34 with the effective image content 36 surrounded by a background image portion 38. In this embodiment, the ef fective image content 36 is orientated parallel to the alignment of the reference points 18, 20 detected by use of the camera 30. For compensation, the effective image content 36 can be shifted without cutting anything away for moderate movement compensation. This might be favorable for working with laptop computers 22 where it would be very annoying if the effective image content 36 being frames of the widows would disappear during compensation.

In an alternative embodiment, the effective image content 36 could have exactly the size of the display 14 and when shifting for compensation the content is cut on one side whereas the other side of the display has a slight unused section (black). This might be favorable for watching videos in environments with small deflections. In another alternative embodiment, the image content 36 could be even larger than the image display 14 and is cut at all edges preferable again with adaptive image content size. When movement compensation is active the image content 36 can be shifted avoiding regions without content to be displayed at. This might be favorable for watching videos in environments with medium to high deflections.

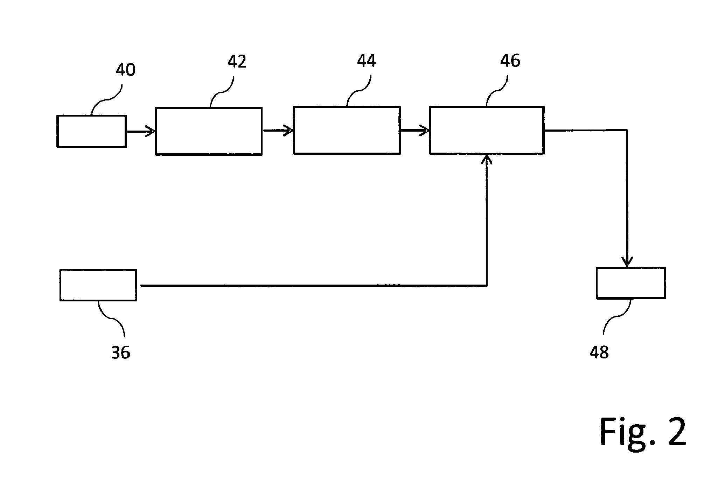

Fig. 2 shows a flow chart illustrating the method for displaying images according to a preferred embodiment of the invention.

Block 40 is representing a step (Step 40) wherein the detecting unit 16 detects the face or faces. In the following step 42, the reference points 18, 20 are detected within the face of the at least one person looking at the image display 14, wherein the detection is performed by use of a face detection algorithm.

In the following step 44, the at least one movement component of a relative movement of the display unit 12 with respect to the reference points 18, 20 is determined.

In the following step 46, the data of movement compensated images 34 for the at least partial compensation of the movement component with respect to the reference points 18, 20 is generated from the image content 36.

Finally, in step 48 the movement compensated images 34 are imaged on the image display 14.

While the invention has been illustrated and described in detail in the drawings and foregoing description, such illustration and description are to be considered illustrative or exemplary and not restrictive; the invention is not limited to the disclosed embodiments.

Other variations to be disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting scope.

## Claims

1. A method for displaying images on an image display (14) of a display unit (12), the method comprising the steps:
- detecting a plurality of reference points (18, 20) within the face of at least one person looking at the image display (14), wherein the detection is performed by use of a detecting unit (16) rigidly coupled to the display unit (12);
- determining at least one movement component of a relative movement of the display unit (12) with respect to the reference points (18, 20);
- generating data of movement compensated images (34) for an at least partial compensation of the movement component with respect to the reference points (18, 20); and
- imaging the movement compensated images (34) on the image display (14).

2. The method according to claim 1, wherein the movement component is a movement component in a direction within a plane parallel to the screen plane of the image display (14).

3. The method according to claim 1 or 2, wherein the movement component or at least one of the movement components is a periodically changing movement component.

4. The method according to claim 3, wherein the imaging of the movement compensated images (34) at least compensates a fundamental frequency portion of the periodically changing movement component.

5. The method according to one of the preceding claims, wherein the movement component or at least one of the movement components is a sudden relative dislocation between the display device and the user.

6. The method according to one of the preceding claims, wherein a periodic or non-periodic movement perpendicular to the screen plane is detected by a reduced distance between the detected reference points (18, 20).

7. The method according to one of the preceding claims, wherein the detecting unit (16) comprises at least one camera (30).

8. The method according to one of the preceding claims, wherein an algorithm for face detection is used for detecting the reference points (18, 20) of the face.

9. The method according to one of the preceding claims, wherein each movement compensated image (34) is composed of the effective image content (36) framed by a background image portion (38) when imaged on the image display (14).

10. The method according to claim 9, wherein each effective image content (36) of the movement compensated images (34) uses a fraction of 80% or less than 80% of the area of the total visible image display content.

11. The method according to one of the preceding claims, wherein the relative movement is a mean relative movement, when the reference points (18, 20) of a plurality of persons are detected.

12. A computer program product comprising a computer usable medium including computer usable program code, wherein the computer usable program code is adapted to execute the method of one of the preceding method claims.

13. A device (10), especially a device (10) for performing the method according to one of the preceding method claims, comprising:
- a display unit (12) with an image display (14) for displaying images,
- a detecting unit (16) suitable for detecting a plurality of reference points (18, 20) within the face of at least one person looking at the image display (14) and
- a processing unit connected to the display unit (12) as well as the detecting unit (16) by signal connection,
wherein the detecting unit (16) is rigidly coupled to the display unit (12),
**characterized in that** the processing unit is arranged for determining at least one movement component of a relative movement of the display unit (12) with respect to the detected reference points (18, 20) and for generating data of movement compensated images (34) for an at least partial compensation of the movement component with respect to the reference points (18, 20).

14. The device according to claim 13, wherein the processing unit is arranged for generating data of movement compensated images (34) for an at least partial compensation of the movement component with respect to the reference points (18, 20).

15. The device according to claim 13 or 14, wherein the device is a handheld device or a laptop device (22).
